(19) Europäisches Patentamt

European Patent Office

Office européen des brevets

(11) EP 0 682 533 B1

(12) **FASCICULE DE BREVET EUROPEEN**

(45) Date de publication et mention
de la délivrance du brevet:
**29.12.1999 Bulletin 1999/52**

(21) Numéro de dépôt: **94906246.7**

(22) Date de dépôt: **04.02.1994**

(51) Int. Cl.$^6$: **A61L 25/00**, A61L 27/00,
A61L 31/00

(86) Numéro de dépôt international:
**PCT/FR94/00135**

(87) Numéro de publication internationale:
**WO 94/17838 (18.08.1994 Gazette 1994/19)**

(54) **MATERIAU SEMI-SYNTHETIQUE UTILISABLE EN MEDECINE ET CHIRURGIE**

ZUR VERWENDUNG IN MEDIZIN UND CHIRURGIE GEEIGNETES HALBSYNTHETISCHES MATERIAL

SEMI-SYNTHETIC MATERIAL FOR MEDICAL AND SURGICAL USE

(84) Etats contractants désignés:
**AT DE DK ES FR GB LU NL PT**

(30) Priorité: **05.02.1993 FR 9301387**

(43) Date de publication de la demande:
**22.11.1995 Bulletin 1995/47**

(73) Titulaire:
**FORTUNE BASE MANAGEMENT, LTD.**
**Central Hong Kong (HK)**

(72) Inventeurs:
 • **Camprasse, Georges**
  **77480 Villenauxe-la-Petite (FR)**
 • **Camprasse, Serge**
  **77500 Chelles (FR)**

(74) Mandataire: **Thinat, Michel et al**
**Cabinet Weinstein,**
**56 A, rue du Faubourg Saint-Honoré**
**75008 Paris (FR)**

(56) Documents cités:
EP-A- 0 395 187        EP-A- 0 532 421
WO-A-90/14111        FR-A- 2 637 502

 • **COMPTES RENDUS DE L'ACADéMIE DES SCIENCES vol. 309-III, no. 6 , 27 Juillet 1989 pages 203 - 210 E. LOPEZ E.A. 'Soudure sans transition (ostéoassimilation) entre l'os maxillaire humain et un implant dentaire compact en calcite naturelle d'Invertébrés marins'**

## Description

La présente invention concerne un produit semi synthétique, obtenu après traitement spécifique, mécanique, thermique et chimique de la nacre, destiné à en modifier la structure et les propriétés de telle manière qu'il puisse être utilisé pour la fabrication de pièces profilées pour la chirurgie orthopédique, cranio-faciale, pour la préparation de ciments biologiques de scellement de prothèses, de matériau de régénération osseuse et de stabilisation en carcinologie et en imagerie interventionnelle.

Le matériau de base, la nacre, provient du test nacré de mollusques aquatiques dont la structure physico-chimique comporte, outre une fraction minérale, représentant 90% à 98% de la masse totale, sous forme de biocristaux, une fraction organique, composée de protéines fibreuses et non fibreuses, solubles et non solubles, possédant une spécificité génique rendant impropre son utilisation en l'état, chez l'animal et chez l'homme.

En effet, le biomatériau utilisé tel quel, se comporterait comme une xénogreffe, et manifesterait dans un temps variable, son caractère antigènique avec réaction immunologique défavorable qui en provoquerait le rejet.

On a trouvé un procédé mécanique, thermique et chimique qui permet d'insolubiliser la fraction soluble des protéines, d'en tanner la partie superficielle et d'empêcher les manifestations tissulaires de rejet.

De préférence, la nacre provient du test nacré de mollusques aquatiques, bivalves, céphalopodes et gastropodes tels que les Pintada (Maxima, Margaritifera) la Conque, le Buccin, le Nautile, l'Ormeau ou tout autre mollusque dont le test contient de la nacre.

Le traitement spécifique du produit selon l'invention se décompose de la façon suivante :

- après préparation mécanique de la coquille du mollusque, afin d'en obtenir la partie nacrée seule, celle-ci est mise à tremper pendant 24 heures dans un bain d'eau déminéralisée contenant 20% d'hypochlorité. Après lavage sous courant d'eau pendant 48 heures, la nacre est portée à ébullition dans un bain d'eau déminéralisée contenant 20% d'hypochlorite de sodium pendant 2 fois 2 heures après changement du bain à chaque opération. Au terme de ces 2 opérations le matériau est rincé, à l'eau bouillante et placé dans une étuve où il subit un traitement à la vapeur d'eau déminéralisée sous pression de 2 bars, pendant 2 fois 2 heures, en présence d'agents chimiques dont l'action est destinée à fixer et tanner la fraction organique et à provoquer une réaction chimique de surface non seulement avec la fraction organique, mais aussi avec la fraction minérale ; les agents sont, par exemple, sans que la liste soit limitative : l'hypochlorite de sodium, l'acide acétique, le glutaraldéhyde, le chlorure de benzalkonium, le formaldéhyde, dilués à 20%.

Au terme de ces opérations le produit selon l'invention est traité à la vapeur d'eau déminéralisée sous pression de 2 bars pendant 1 heure, sans adjonction de produit chimique. Le produit selon l'invention est séché, sous vide, par paliers thermiques, jusqu'à 100°.

A partir de là, le matériau selon l'invention peut être stérilisé par rayonnement gamma à 2,5 mégarad, et se trouve prêt à l'emploi.

Afin de modifier les propriétés fonctionnelles du matériau sous forme pulvérulente ou plastique, on peut lui adjoindre des composés minéraux tels : l'hydroxyde de calcium, le phosphate de calcium, le chlorure de calcium ou tout autre sel de calcium, le produit selon l'invention sous sa forme pulvérulente ou plastique possède dès lors des propriétés adhésives qui le rende apte à être associé à d'autres matériaux tels que, par exemple, les métaux, les matières plastiques.

Le produit selon l'invention peut être aussi associé à des monomères ou des polymères ainsi qu'à des dérivés méthyliques ou acryliques.

Le produirt selon l'invention tant sous sa forme compacte que pulvérulente, peut être imprégné sous pression, de produits médicamenteux se fixant sur la fraction organique, et destinés à être relargués "in situ" à doses thérapeutiques, pendant un temps déterminé pour le traitement de certaines affections touchant aussi bien le tissu osseux que les organes creux.

Dans un mode de réalisation préférentielle le produit selon l'invention est utilisé comme stérilet imprégné de substances médicamenteuses pour le traitement d'affections utérines ou de substances contraceptives.

Le produit selon l'invention peut servir de support de greffe de cristallin après épithélialisation de sa surface, sous la muqueuse jugale.

Il peut être usiné sous forme d'éléments de substitution de la chaîne des osselets de l'oreille moyenne.

On sait que lors du traitement physico-chimique de la nacre il s'y produit un certain nombre de réactions chimiques de surface dont l'une d'entre elles se matérialise de la façon suivante :

$$CaCO_3 + 2Na + 2HclO \xrightarrow{T^o, P} Ca(0H)_2 + Cl_2 + 2Na^+ + Co_32-$$

Le produit de surface obtenu est un précipité stable caractérisant le produit selon l'invention.

Lorsque le produit selon l'invention est placé dans le tissu osseux, sous l'effet de la pression osmotique et en présence des gaz du sang, notamment le $Co_2$ et l'oxygène $O_2$ il se produit entre autres, la réaction chimique suivante :

$$Ca(OH)_2 + Co_2 + O_2 \rightarrow CaCo_3 + H_2O + O_2$$

Cette réaction résultant des propriétés fonctionnelles du matériau selon l'invention et découlant du traitement de celui-ci, explique, en partie, les propriétés bioactives, du produit selon l'invention.

Le produit selon l'invention peut être utilisé seul, ou associé à des anti-mitotiques ou et à des antibiotiques, et inséré ou injecté à l'aide de vecteurs dans des cavités ou des pertes de substances osseuses d'origines carcinologiques afin d'en consolider la structure et d'en stabiliser l'évolution.

Selon un mode de réalisation préférentielle le produit selon l'invention sous sa forme compacte, ou sous forme pulvérulente associée à des monomères ou des polymères peut être utilisé comme matériau d'embolisation dans les tumeurs d'origines vasculaires tels naevus, hémangiomes, sans que cette liste soit limitative.

Le produit selon l'invention peut être utilisé, sous sa forme pulvérulente, comme matériau de consolidation dans les géodes de la tête fémorale et les maladies dégénératives du rachis.

L'exemple suivant illustre le comportement du matériau selon l'invention utilisé en chirurgie. Des implants cylindriques de 10 mm de long et de 4 mm de diamètre ont été placés dans des cavités adéquates situées dans le fémur du chien beagle. Les uns ont été réalisés à partir de nacre décontaminée et stérilisée, les autres ont été réalisés à partir du matériau selon l'invention obtenu après traitement spécifique.

Dans les premiers cas on a observé dans des délais variables qui n'excédaient pas 30 jours, des réactions immunologiques qui ont abouti à l'expulsion de tous les implants.

Dans le deuxième cas pour tous les implants réalisés dans le matériau selon l'invention on a noté radiologiquement l'absence de zone radioclaire entre l'os receveur et les implants, cliniquement une ostéointégration parfaite entre l'os receveur et les implants matérialisés par une rigidité de ces derniers, histologiquement par la mise en évidence d'une brasure existant à l'interface os-implant.

On peut considérer comme particulièrement intéressant le comportement du matériau selon l'invention qui, sous forme compacte, réalise une fusion physico-chimique avec l'os receveur et une fois l'ostéointégration achevée ne subit aucune altération structurelle dans le temps, alors que sous sa forme pulvérulente plastique ou combinée, il présente non seulement des propriétés adhésives avec l'os ou les métaux ou tous autres matériaux, mais encore des propriétés hémostatiques et eutrophiques, matérialisées par un arrêt des suffusions hémorragiques lors de sa mise en place sur le site opératoire et une cicatrisation muqueuse accélérée. En outre, il provoque des réactions enzymatiques locales qui conduisent à sa transformation totale en tissu osseux.

Les descriptions suivantes illustrent l'utilisation du matériau selon l'invention sans que ces exemples soient limitatifs, il appartient à l'homme de métier de meure en oeuvre le matériau selon l'invention toutes les fois où il aura besoin de prothèse orthopédiques ou de ciment biologique ou de toutes autres utilisations en chirurgie ou médecine animale et humaine.

Le matériau selon l'invention sous sa forme compacte peut prendre la forme d'un plateau tibial, de condyle fémoral, de cavité cotyloîde, de l'articulation dela hanche, de tête humérale, d'aile illiaque, d'os de la voute cranienne, de vis. Il peut, sous sa forme pulvérulente, combinée au collagène ou autres, et séchée, consituer des chevilles de fusion intervertébrale, des coins d'ostéotomie d'ouverture, de cales intersomatiques, des bouchons de cavités de trépanation osseuse, kystiques ou tumorales.

L'exemple suivant illustre l'utilisation du produit selon l'invention sous forme de ciment biologique de scellement dans le cas d'une arthroplastie totale de la hanche.

Depuis plus de 20 ans les prothsèes de hanches sont scellées par des ciments chirurgicaux dont le plus utilisé est le métacrylate de méthyle, matériau plastique qui en durçissant réalise un ancrage entre le métal et la corticale osseuse du fémur dans lequel est implantée la prothèse. Plus récemment on a proposé des prothèses métalliques conçues de façon à réaliser un ancrage biologique sans ciment. Dans le cas des ciments acryliques, plusieurs auteurs ont attiré l'attention sur les inconvénients de ces derniers : diffusion du monomère dans la circulation, propriétés mécaniques médiocres, manque d'élasticité, vieillissement . Mais l'inconvénient majeur réside dans le fait que le durcissement lors de la prise s'accompagne d'une réaction exothermique et chimique provoquant dans un nombre non négligeable de cas une nécrose osseuse intéréssant la corticale fémorale, ce qui aboutit à la mobilité de la prothèse, augmenté d'un risque infectieux nécessitant l'éradication du ciment et une arthroplastie de reprise.

Le matériau selon l'invention sous sa forme pulvérulente a été utilisé lors d'une arthroplastie totale de la hanche vierge pour coxarthrose avec pose d'une prothèse à appui trochantéro diaphysaire. Le ciment de scellement est réalisé extamporanément avec 20g du produit selon l'invention sous sa forme pulvérulente auquel on ajoute de manière fractionné de sang total provenant du patient de façon à obtenir une pâte de consistance crémeuse. Le fémur est préparé par résection de la tête à la base du col au dessus du petit trochanter à l'aplomb interne du grand trochanter à l'aide d'une scie oscillante. Le cotyle est préparé pour recevoir le composant acétabulaire composé de la cupule cotyloîdienne. Le canal médullaire est ajusté par des rapes fantômes de l'implant et rempli par le ciment biologique préalablement préparé. La queue de la prothèse est également enduite de ciment et son implantation est faite à friction modérée. Les critéres de Merle d'Aubigné appliqués à l'exemple cité plus haut mettent en évidence l'excellence du résultat de l'utilisation du produit selon l'invention sous forme pulvérulente : indolence, stabilité de l'appui unipodal.

L'examen radiologique pratiqué à un an met en évidence une ossification du ciment biologique préparé à partir du produit selon l'invention sans image radiocalire entre le pivot de l'implant et l'os néoformé dans le canal médulaire, témoignant d'une indiscutable stabilité de la prothèse. Il est à noter que les phénomènes de minéralisation de la pâte formée du produit selon l'invention et du sang du patient apparaissent dès les premières semaines et la formation du tissu osseux néoformé est complète à un an.

Un autre mode d'utilisation du produit selon l'invention est illustré par l'exemple ci-dessous.

On sait que lors de la réalisation d'arthrodèse vertébrale, le greffon autogène est prélevé sur la crête illiaque intéressant quelques fois les lèvres interne et externe ainsi que les faces interne et externe de la partie supérieure de l'aile illiaque ce qui représente une perte de substance importante, aggravée par la possibilité d'une nécrose dûe à la lésion d'une artère nourricière dans la zone du greffon. Dans l'exemple étudié, la prise du greffon à provoqué un défect osseux de 60mm de longueur et de 45 mm de hauteur, accompagné de douleurs et d'une impotence fonctionnelle par désinsertions de certains faisceaux du grand droit de l'abdomen. du tranverse, du petit oblique et des faisceaux superficiels du petit fessier. Un fragment du matériau selon l'invention est taillé aux dimensions de la perte de substance avec un épaulement sur le pourtour percé de pertuis pour l'insertion des tendons des muscles désinsérés et la fixation sur l'aile illiaque.

La pièce orthopédique après traitement, décontamination et stérilisation au rayonnement gamma à 2.5 mgrd, est insérée de telle manière que la pression des viscères la plaque contre l'aile illiaque, fixée au moyen de vis taillées dans le matériau selon l'invention. Les faisceaux des muscles et aponévroses sont réinsérés aux moyens de ligatures non résorbables.

Les suites opératoires sont normales. L'examen clinique montre une parfaite réinsertion des faisceaux musculaires, l'examen radiologique une parfaite ostéointégration du matériau selon l'invention sans aucune manifestations inflammatoire ou immunologique de rejet.

Ces observations et expérimentations montrent :

a) que le produit selon l'invention est parfaitement biocompatible quelque soit sa forme.
b) qu'il n'entraine aucune manifestation systémique de rejet lors de son utilisation.
c) que le traitement du produit selon l'invention lui confère des qualités fonctionnelles nouvelles qui étendent son champ d'application.
d) que le produit selon l'invention sous forme pulvérulente ou plastique présente des propriétés adhésives, hémostatiques.

**Revendications**

1.    Produit set-synthétique destiné à la réalisation de pièces profilées utilisées en chirurgie orthopédique, de matériau de régénération et de stabilisation osseuse, de ciment de scellement biologique, caractérisé en ce qu'il est fabriqué à partir de la nacre de mollusques aquatiques subissant un traitement comprenant les étapes de :

-    trempage dans un bain d'eau déminéralisée contenant 20% d'hypochlorite, la solution étant portée à ébullition pendant 2 heures,

-    changement du bain et nouveau trempage dans une solution d'eau déminéralisée contenant 20% d'hypochlorite de sodium, la solution étant portée à ébullition pendant 2 heures,

-    traitement par de la vapeur d'eau déminéralisée, additionnée d'hypochlorite de sodium à 20% ou de tout autre agent chimique tel que l'acide acétique, le chlorure de benzalkonium, le formaldéhyde, dilué à 20% sous pression de 2 bars pendant deux fois 2 heures,

-    traitement par de la vapeur d'eau déminéralisée seule, sous pression de 2 bars pendant 1 heure, et

-    séchage sous vide par paliers thermiques jusqu'à 100°C.

2.    Produit selon la revendication 1, caractérisé en ce qu'il est réalisé à partir de la nacre de Pinctada Maxima, de Pinctada Margaritifera, de Tricdane, de Conque, de Buccin, de Triton, de Nautile, d'Ormeaux.

3. Produit selon la revendication 1 ou 2, caractérisé en ce qu'il est réduit à l'état pulvérulent avec une granulométrie allant de 1 à 100 μm.

4. Produit selon l'une quelconque des revendications précédentes, caractérisé en ce qu'il est mélangé sous forme pulvérulente à l'hydroxyde de calcium, le phosphate de calcium, le chlorure de calcium ou tout autre sel de calcium.

5. Produit selon l'une quelconque des revendications précédentes, caractérisé en ce qu'il est sous forme pulvérulente, associé au collagène, d'origine animale ou humaine, à de l'amidon, de façon extemporanée.

6. Produit selon l'une quelconque des revendications précédentes à utiliser en ce qu'il est associé, sous forme pulvérulente, à du collagène, d'origine animale ou humaine, à de l'amidon, puis séché et moulé en forme d'élements prothétiques utilisables en chirurgie.

7. Produit selon l'une quelconque des revendications précédentes, caractérisé en ce qu'il est associé à des monomères ou des polymères ainsi qu'à des dérivés méthyliques ou acryliques, des antibiotiques ou des antimitotiques.

8. Utilisation du produit selon l'une quelconque des revendications précédentes, pour la réalisation d'implants, de pièces profilées en chirurgie orthopédique, de coins d'ostéotomie, de cales intersomatiques, de vis et plaques d'ostéosynthèse, de support pour greffe de cristallin, de stérilet, de substitut de la chaîne des osselets de l'oreille moyenne.

9. Utilisation du produit selon l'une des revendications 1 à 7 comme revêtement de prothèses orthopédiques métalliques ou plastiques, par tout procédé physique ou chimique.

10. Utilisation du produit selon l'une quelconque des revendications 1 à 7 comme revêtement de prothèses tendineuses ou ligamentaires.

11. Utilisation du produit selon l'une quelconque des revendications 1 à 7 en chirurgie orthopédique, chirurgie maxillo facile, odontostomatologie, chirurgie carcinologique et traumatologique, otorhinolaryngologie, gynécologie, ophtalmologie.

## Claims

1. A semi-synthetic product intended to provide shaped pieces used in orthopedic surgery, for bony regeneration and stabilization material, and as a biological sealing cement, characterized in that the product is fabricated from mother of pearl of aquatic mollusks which has undergone a treatment comprising the steps of:

   - soaking in a bath of demineralized water containing 20% of hypochlorite, while boiling the solution for 2 hours,
   - changing the bath and soaking in a new solution of demineralized water containing 20% of sodium hypochlorite, while boiling the solution for 2 hours,
   - treating with demineralized water vapor, admixed with 20% sodium hypochlorite or with another chemical agent such as acetic acid, benzalkonium chloride, or formaldehyde, diluted at 20% under a pressure of 2 bars for 2 periods of 2 hours,
   - treating with demineralized water vapor alone, under a pressure of 2 bars for a period of 1 hour, and
   - drying under vacuum in thermal stages up to 100° C.

2. The product of claim 1, characterized in that the product is made from the mother of pearl of Pinctada Maxima, of Pinctada Margaritifera, of Tricdane, of Concha, of Whelk, of Triton, of Nautilus, of Ormers.

3. The product of any one of claims 1 or 2, characterized in that the product is reduced to a powdery state having a particle size of 1 to 100 μm.

4. The product according to any of the preceding claims, characterized in that the product is mixed in powdery form with calcium hydroxide, calcium phosphate, calcium chloride or any other calcium salt.

5. The product according to any one of the preceding claims, characterized in that the product in a powdery form is associated with collagen of human or animal origin, with starch in an extemporaneous manner.

6. The product according to any preceding claim to be used when it is associated in powdery form with collagen of human or animal origin, with starch, and then dried and molded in the shape of prosthetic elements usable in surgery.

7. The product according to any of the preceding claims, characterized in that the product is associated with monomers or polymers as well as with methylic or acrylic derivatives, antibiotic or antimitotic substances.

8. Use of the product according to any of the preceding claims for the production of implants, of shaped pieces for orthopedic surgery, for osteotomy wedges, for intersomatic blocks, for screws and plates for osteosynthesis, as a support for the graft of crystalline lens, for birth control coil, and as a substitute for the chain of ossicles of the middle ear.

9. Use of the product according to any one of claims 1-7 as a coating for metal or plastic orthopedic prosthesis during a physical or chemical process.

10. Use of the product according to any one of claims 1-7 as a coating for tendon or ligament prosthesis.

11. Use of the product according to any one of claims 1-7 in orthopedic surgery, maxillo facial surgery, odontostomatology, carcinological and traumatological surgery, otorhinolaryngology, gynecology, and ophthalmology.

**Patentansprüche**

1. Halb-synthetisches Produkt für die Herstellung von Profilstücken aus biologischem Dichtungszement zur Anwendung in der orthopädischen Chirurgie zur Regeneration und Stabilisierung von Knochen, dadurch gekennzeichnet, dass es auf der Basis von Perlmutter von aquatischen Weichtieren hergestellt wird, welche einer Behandlung mit den folgenden Schritten unterworfen werden:

   - Einweichen in einem 20% Hypochlorit enthaltenden entmineralisierten Wasserbad, wobei die Lösung zwei Stunden gekocht wird,
   - Wechsel des Bades und erneutes Einweichen in einer 20% Natriumhypochlorit enthaltenden entmineralisierten Wasserlösung, wobei die Lösung zwei Stunden gekocht wird,
   - Behandlung mit entmineralisiertem Wasserdampf, dem 20% Natriumhypochlorit und andere chemische Wirkstoffe, beispielsweise Essigsäure, Benzalkoniumchlorid, Formaldehyd, zugesetzt werden, und Verdünnung unter einem Druck von 2 bar während zweimal zwei Stunden,
   - Behandlung nur mit entmineralisiertem Wasserdampf unter einem Druck von 2 bar während 1 Stunde, und
   - Trocknen bei Unterdruck in Heizschritten bis zu 100°C.

2. Produkt nach Anspruch 1, dadurch gekennzeichnet, dass es auf der Basis von Perlmutter der Pinctada Maxima, Pinctada Margaritifera, Triodane, Trompetenschnecke, Wellhornschnecke, Nautilusmollusk, Haliotismollusk hergestellt ist.

3. Produkt nach Anspruch 1 oder 2, dadurch gekennzeichnet, dass es in Pulverform mit einer Korngrössenabstufung zwischen 1 und 100 μm überführt wird.

4. Produkt nach einem der vorhergehenden Ansprüche, dadurch gekennzeichnet, dass es in Pulverform mit Calciumhydroxid, Calciumphosphat, Calciumchlorid, oder anderen Calciumsalzen gemischt wird.

5. Produkt nach einem der vorhergehenden Ansprüche, dadurch gekennzeichnet, dass es in Pulverform unvorbereitet mit aus Stärke hergestelltem tierischem oder menschlichem Kollagen assoziiert wird.

6. Produkt nach einem der vorhergehenden Ansprüche, welches in Pulverform mit aus Stärke hergestelltem tierischem oder menschlichem Kollagen assoziiert wird, und dann getrocknet und in der Form von prosthetischen Elementen, die in der Chirurgie brauchbar sind, angewandt wird.

7. Produkt nach einem der vorhergehenden Ansprüche, dadurch gekennzeichnet, dass es sowohl mit Momoneren und Polymeren, als auch mit methylitischen oder acrylitischen Derivaten, Antibiotika oder Antimitotika asoziiert ist.

8. Verwendung des Produktes nach einem der vorhergehenden Ansprüche zur Herstellung von Implantaten, profilierten Elementen für die orthopädischen Chirurgie, osteotomischen Polstern, intersomatischen Polstern, osteosynthetische Schrauben und Platten, zur Unterstützung von Implantaten von Kristallinsen, Empfängnissverhütungsspiralen, als Ersatz für die Kette der Gehörknöchelchen im Mittelohr.

9. Verwendung des Produktes nach einem der Ansprüche 1 bis 7 als Belag für Prothesen aus Metall oder Kunststoff für alle physikalischen oder chemischen Verfahren.

10. Verwendung des Produktes nach einem der Ansprüche 1 bis 7 als Belag für Sehnen- oder Ligamentprothesen.

11. Verwendung des Produktes nach einem der Ansprüche 1 bis 7 in der orthopädischen Chirurgie, maxillo-fazialen Chirurgie, Ondontostomatologie, Krebs- und traumatologischen Chirurgie, Otorhinolarygologie, Gynäkologie, Ophtalmology.